# EUROPEAN PATENT APPLICATION

(11) **EP 4 678 091 A1**
(43) Date of publication of application: **14.01.2026**
(21) Application number: 24198054.9
(22) Date of filing: 03.09.2024
(51) Int. Cl.: A61B 5/00, G02B 27/01, G06F 3/01

(54) **SYSTEM AND METHOD FOR MONITORING HEALTH STATUS**

(30) Priority: 09.07.2024 PL 44915924
(71) Applicant: Akademia Gorniczo-Hutnicza im. Stanislawa Staszica w Krakowie, 30-059 Krakow (PL)
(72) Inventor: Hemmerling, Daria, 31-216 Kraków (PL); Daniol, Mateusz, 31-334 Kraków (PL); Skalski, Andrzej, 31-091 Kozierów (PL); Wodzinski, Marek, 32-080 Zabierzów (PL); Kaminski, Jakub, 30-611 Kraków (PL); Kaczmarska, Marta, 31-213 Kraków (PL); Wójcik-Pedziwiatr, Magdalena, 31-572 Kraków (PL); Jemiolo, Pawe, 30-011 Kraków (PL)
(74) Representative: Rybarczyk, Dariusz Pawel

(57) **Abstract**

The invention relates to a system for monitoring health status of an examined person, comprising goggles designed to be mounted on the head of the examined person and comprising a transparent display positioned in the field of view of the examined person, adapted to enable simultaneous observation of the surroundings and displaying predetermined augmented reality virtual content at different depth levels; a processing unit connected to an internal memory; one or more sensors selected from a first group of sensors integrated with the goggles for monitoring physical and/or behavioral parameters of the examined person; and at least one communication interface. The system is characterized in that it further comprises one or more sensors selected from a second group of sensors for monitoring physical and/or behavioral parameters of the examined person, arranged on or near the body of the examined person, communicatively connected to the goggles by means of said at least one communication interface, the sensors of the second group being independent of the sensors of the first group. The goggles are in particular augmented reality (AR), mixed reality (MR) or virtual reality (VR) goggles and are configured to provide predetermined instructions or stimuli to the examined person, to collect and store sensor signals from the first group of sensors and the second group of sensors in the internal memory, reflecting the response to the instructions or stimuli, and to jointly process and multimodally analyze them using the processing unit in order to assess the health status of the examined person.

The invention further relates to a method for monitoring health status of an examined person, which may be implemented in the system according to the invention, and comprises jointly analyzing sensor data recorded by the first and second groups of sensors.

## Description

The present invention relates to a system and method for monitoring the health status of an examined person, and in particular to a system and method that use augmented reality goggles placed on the head of an examined person and a series of sensors for measuring and processing human vital and behavioral signals of the examined person for the purpose of ongoing monitoring of the health status.

The state of human health, both physical and mental, requires regular check-ups to detect as quickly as possible any irregularities or anomalies that may indicate a deteriorating state of health, diseases or other factors that threaten health. Early detection of health threats is a key factor in determining the effectiveness of the treatment or therapy undertaken. In many cases, a diagnosis made too late reduces the chances of taking effective action and cure. For this reason, there is a need for solutions that enable ongoing monitoring of important vital and behavioral parameters and their fast and accurate analysis in order to detect any health threats.

In the state of the art, a number of solutions have been developed to enable monitoring of the health status.

EP3472828B1 describes an augmented reality display system with inwardly-directed sensors (towards the user). The system is intended to perform neurological analysis by determining a response to a stimulus by receiving data from at least one inwardly-directed sensor and identifying a neurological abnormality or deficiency associated with the response.

US11612341B2 describes a virtual or augmented reality based system for assessment and measurement of reaction time. In various embodiments the system, methods, and computer program products relate to assessing and measuring a reaction time of a patient/user in virtual reality (VR) or augmented reality (AR) environments. The VR/AR system may provide a sensory stimulus to the patient/user in the VR/AR environment, determine a plurality of motion parameters, and determine a reaction time based on the plurality of motion parameters by applying a time window selection model.

WO201928268A1 describes a virtual or augmented reality based tracking system for assessment of postural sway. In various embodiments, positional data are collected from a user from a goggles and/or one or more hand sensors. The positional data is processed to determine the center of mass of the user. In determining of the center of mass, positional data may be given a higher or lower weight compared to other positional data. A virtual reality environment is presented to the user through the goggles and may be altered to simulate unbalancing of the user. The raw position data and center of mass are sent to a remote server. A report of the user sway is generated from the center of mass. Nausea reduction may be implemented by narrowing a field of vision of the user.

WO2022/212798 describes a method of detecting neurological impairment and a device for testing sensorimotor control to detect neurological impairment. The method includes positioning a head-mounted display on the user's head, the head-mounted display placing the user in a virtual or augmented reality environment; presenting the subject with a software-generated object in the virtual or augmented reality environment; providing instructions directing the user to execute one or more sensorimotor activities relating to the software-generated object within the virtual or augmented reality environment; recording data during execution of the one or more objectives; and determining if there is a neurological impairment based upon the recorded data. The device includes a head-mounted display, one or more processors, and one or more hardware storage devices having stored thereon computer-executable instructions which are executable by the one or more processors to display a virtual immersive environment in the head-mounted display.

US11559355B2 relates to an augmented and virtual reality for use with neuromodulation therapy.

WO2022/272093 describes a system comprising: a virtual or augmented reality system adapted to provide a virtual environment to a user with; at least one sensor coupled to the user; a computing node comprising a computer readable storage medium having program instructions embodied therewith, the program instructions executable by a processor of the computing node to cause the processor to perform a method comprising: presenting a first visual stimulus to the user within the virtual environment, the first visual stimulus having a high spatial frequency; and presenting a second visual stimulus to the user within the virtual environment, the second visual stimulus having a low spatial frequency. The method comprising: providing a virtual environment to a user via a virtual or augmented reality system; presenting a first visual stimulus to the user within the virtual environment, the first visual stimulus having a high spatial frequency; and presenting a second visual stimulus to the user within the virtual environment, the second visual stimulus having a low spatial frequency.

In the publication "Design, implementation, and metrological characterization of a wearable, integrated AR-BCI hands-free system for health 4.0 monitoring", Measurement, Elsevier, DOI: 10.1016/j.measurement.2021.109280, an integrated real-time monitoring system based on Augmented Reality (AR) and Brain-Computer interface (BCI) for hands-free acquisition and visualization of remote data is proposed. As a case study, the monitoring of patients' vitals in the operating room is considered; in particular, through the suitable combination of BCI and AR, the anesthesiologist can monitor in real-time (through a set of AR glasses) the patient's vitals acquired from the electromedical equipment.

"Digital biomarkers for Alzheimer's disease: the mobile/ wearable devices opportunity", NPJ Digital Medicine, DOI: https://doi.org/10.1038/s41746-019-0084-2, addresses the topic of identification and use of readily available digital biomarkers, which harness advances in mobile and wearable technologies. Topics examined include a review of existing early clinical manifestations of AD and a path to respective sensor and mobile/wearable device usage to acquire domain-centric data towards objective, high frequency and passive data phenotyping.

Taking into account the developed solutions and the problem identified at the beginning, further improvements are still needed in solutions enabling health monitoring with the simultaneous use of multiple sensors and augmented reality.

Accordingly, the invention relates to a system and method for monitoring the health of an examined person as defined in the independent claims. The dependent claims indicate advantageous variants of the invention.

According to the invention, there is provided a system for monitoring health status comprising goggles designed to be mounted on the head of an examined person and comprising a transparent display positioned in the field of view of the examined person, adapted to enable simultaneous observation of the surroundings and displaying predetermined augmented reality virtual content at different depth levels; a processing unit connected to an internal memory; one or more sensors selected from a first group of sensors integrated with the goggles for monitoring physical and/or behavioral parameters of the examined person. The system further comprises at least one communication interface and is characterized in that it further comprises one or more sensors selected from a second group of sensors for monitoring physical and/or behavioral parameters of the examined person, arranged on or near the body of the examined person, communicatively connected to the goggles by means of said at least one communication interface, the sensors of the second group being independent of the sensors of the first group. The goggles are in particular augmented reality (AR), mixed reality (MR) or virtual reality (VR) goggles and are configured to provide predetermined instructions or stimuli to the examined person, to collect and store sensor signals from the first group of sensors and the second group of sensors in the internal memory, reflecting the response to the instructions or stimuli, and to jointly process and multimodally analyze them using the processing unit in order to assess the health status of the examined person.

In one preferred embodiment of the system, the first group of sensors integrated in the goggles comprises in particular RGB cameras directed at the environment around the examined person, a microphone or microphone array, infrared cameras directed at the eyes of the examined person; a heart rate sensor; a blood pressure monitor; a pulse oximeter; an electrodermal activity sensor; an accelerometer, a gyroscope and a magnetometer; an eye movement sensor; a thermometer; depth cameras, an infrared (IR) sensor; a near infrared spectroscopy sensor (FNIRS); an electrode array together with an EEG sensor.

In another preferred embodiment of the system, the second group of sensors comprises in particular an ECG sensor, an EEG sensor; an EMG sensor; a GPS module; an EDA sensor; pressure sensors integrated in shoe insoles for measuring foot pressure on the ground and gait analysis; electrodes placed on the neck for examining vocal folds, a blood pressure sensor, a heart rate sensor (PPG), a light sensor, a temperature sensor, an acoustic signal sensor, an accelerometer, a gyroscope, a magnetometer, a blood oxygen saturation sensor.

In yet another preferred embodiment of the system, the goggles are additionally equipped with at least one speaker or headphones.

In yet another preferred embodiment of the system, the display of the goggles is configured to provide instructions or stimuli in a visual form and/or the speaker or headphones are configured to provide instructions or stimuli in an audible form, wherein the visual instructions or stimuli are in the form of one or more images at different depth levels.

In yet another preferred embodiment, the system is configured to recognize and monitor neurological or neurodegenerative diseases, wherein the sensors used from the first group used include: IR cameras for tracking eye movement, at least one microphone for recording speech, an accelerometer, a gyroscope and a magnetometer for measuring posture and monitoring gait, RGB cameras for tracking upper limb movement and depth cameras for tracking facial movements and expressions, and the sensors used from the second group include an EEG sensor; an EMG sensor; a GPS module; an EDA sensor; pressure sensors integrated in shoe insoles for measuring foot pressure on the ground and gait analysis; electrodes placed on the neck for examining vocal folds, a blood pressure sensor, a heart rate sensor (PPG), a temperature sensor, and a blood oxygen saturation sensor.

In yet another preferred embodiment, the system comprises at least one external workstation communicatively connected to the goggles via said communication interface, in particular a computer or a smartphone, for receiving, processing and/or collecting sensor data and/or signals.

The invention further relates to a method for monitoring health status of an examined person, which is characterized by mounting, on the head of the examined person, goggles equipped with a transparent display, in particular augmented reality (AR), mixed reality (MR) or virtual reality (VR) goggles, and equipped with one or more integrated sensors selected from a first group of sensors for monitoring physical and/or behavioral parameters of the examined person, and furthermore, arranging, on the body of the examined person or in its vicinity, one or more sensors selected from a second group of sensors for monitoring physical and/or behavioral parameters of the examined person, the sensors of the second group being independent of the sensors of the first group, and next providing predetermined visual and/or auditory instructions or stimuli to the examined person by means of a transparent display or by means of at least one loudspeaker or headphones; recording, by means of sensors from the first group and sensors from the second group, physiological and/or behavioral parameters reflecting the reaction of the examined person to the provided instructions or stimuli; and jointly analyzing the recorded sensor data. The analyzing includes preprocessing that includes filtering, normalization and segmentation of measurement signals; analysis of time and/or frequency and/or amplitude features of the pre-processed signals in order to extract significant features; detection of patterns and classification of the extracted features; assessment of the health status of the examined person based on the performed classification of features.

In one preferred embodiment of the method, the first group of sensors integrated in the goggles, which are used for recording physiological and/or behavioral parameters of the examined person, comprises in particular RGB cameras directed at the environment around the examined person, a microphone or a microphone array, cameras operating in the infrared range directed at the eyes of the examined person; a heart rate sensor; a blood pressure monitor; a pulse oximeter; an electrodermal activity sensor; an accelerometer, a gyroscope and a magnetometer; an eye movement sensor; a thermometer, depth cameras, an infrared (IR) sensor; a near infrared spectroscopy sensor (FNIRS); an electrode array together with an EEG sensor.

In another advantageous embodiment of the method, the second group of sensors, which are used for recording physiological and/or behavioral parameters of the examined person, comprises in particular an ECG sensor, an EEG sensor; an EMG sensor; a GPS module; an EDA sensor; pressure sensors integrated in shoe insoles for measuring foot pressure on the ground and analyzing gait; electrodes placed on the neck for measuring the vocal folds, a blood pressure sensor, a heart rate sensor (PPG), a light sensor, a temperature sensor, an acoustic signal sensor, an accelerometer, a gyroscope, a magnetometer, and a blood oxygen saturation sensor.

In yet another preferred embodiment of the method, machine learning and artificial intelligence algorithms, in particularl neural networks, are used for detecting patterns and classifying the extracted features.

In yet another preferred embodiment of the method, based on the senosor signals and data collected for a given examined person a model is created using machine learning to personalize and adapt the analysis.

In yet another preferred embodiment of the method, based on the assessment of the health status of the examined person, feedback, health reports or alerts are generated in real time and displayed on the display of the goggles and/or transmitted to one or more external devices.

In yet another preferred embodiment, the method is adapted for monitoring neurological or neurodegenerative diseases, wherein physiological and/or behavioral parameters are recorded using integrated sensors from the first group: eye movement using IR cameras, speech using at least one microphone, posture and gait using an accelerometer, a gyroscope and a magnetometer, movement of the upper limbs using RGB cameras, and facial movements and expressions using depth cameras. Furthermore, physiological and/or behavioral parameters are recorded using sensors from the second group: brain activity using an EEG sensor; muscle and nerve function using an EMG sensor; movement patterns using a GPS sensor; electrodermal activity using an EDA sensor; foot pressure on the ground and gait using pressure sensors integrated in shoe insoles; vocal fold activity using electrodes placed on the neck, blood pressure and using a blood pressure sensor, heart rate using a PPG sensor; body temperature using a temperature sensor; blood oxygen saturation using a blood oxygen saturation sensor. Multimodal analysis includes jointly: analysis of eye movement to detect characteristic movement patterns, in particular nystagmus and/or slowing of eye reactions; speech analysis, in particular tempo, intonation, and voice quality; analysis of posture measurement, in particular changes in body balance and stability; analysis of the examined person's reaction to visual and/or auditory stimuli; analysis of facial expressions; analysis of gait; analysis of electroencephalographic (EEG) signals; analysis of the near-infrared spectroscopy signal carrying diagnostic information regarding active parts of the brain; analysis of the electromyography (EMG) signal carrying information about muscle activity; analysis of signals form electroglottography, which provides information about vocal fold activity; analysis of signals carrying information about systolic and diastolic blood pressure; analysis of the photoplethysmographic signal, providing information about heart rate; analysis of sleep quality based on one or more parameters measured during the sleep of the examined person, including: body movements, heart rate, skin temperature, blood oxygen saturation, ambient light intensity.

The invention further relates to a computer-readable storage medium comprising computer program instructions strored thereon which, when executed by a processing unit, perform the above-described method.

The claimed solution based on the use of augmented reality (AR), wearable sensors, and data analysis has many potential advantages and benefits for patients, medical personnel, and the entire healthcare system. Some of the main advantages of this solution are as follows:
- personalized and objective diagnostics: the solution enables the collection of objective data on the patient's psychophysical condition, which can help in more accurate and personalized diagnostics;
- early detection and monitoring: thanks to systematic monitoring of patients, the system can detect early symptoms or changes in health, which allows for a faster response and avoidance of more serious health problems;
- reduction of human errors: automation of the diagnostic process using data analysis and artificial intelligence minimizes the risk of human errors when interpreting results;
- improvement of patient-medical staff interaction: the solution can facilitate communication between the patient and medical staff by providing objective data that can serve as a reference point in diagnosis and treatment planning;
- continuous monitoring throughout the day: patients are monitored in real time while performing daily activities, so that more comprehensive and natural data than traditional one-time tests is provided;
- optimization of therapy and treatment: based on data analysis, therapies and treatments can be tailored to the specific needs of the patient, increasing efficiency and minimizing side effects;
- clinical research and disease research: the solution can be used in clinical research, allowing scientists and doctors to collect large amounts of medical data in a nearly non-intrusive way;
- optimization of medical resources: thanks to continuous monitoring and diagnostics, it is possible to better manage medical resources and avoid unnecessary visits or hospitalizations;
- improvement of patient education: the solution can provide educational information and guidance to patients in real time, helping them understand their health status and make informed decisions;
- disease prediction and long-term care: data analysis can help create predictive models that predict the risk of developing certain diseases or health problems in the future;
- remote medical care: the system enables remote monitoring of patients, which can be particularly valuable for people living in remote locations or requiring constant care;
- continuous improvement and innovation: the system provides flexibility and the possibility of continuous improvement through updates to the user interface, analysis algorithms, and integration of new sensors or technologies.

The novelty and contribution of the invention lies in an advanced health monitoring system, in particular in the field of neurology, which integrates augmented reality (AR) technology with a comprehensive set of internal and external sensors. The main innovation lies in the use of AR goggles, which not only display visual content at different depth levels, but also use built-in and external sensors to continuously monitor the user's neurological parameters. Data integration allows precise analysis of the neurological health status in real time. Thanks to advanced machine learning algorithms, the system is able to detect subtle changes and anomalies in signals, which allows early detection and monitoring of the examined person's neurological conditions. In addition, the ability to communicate with the external sensors using wireless technology expands the range of monitored parameters, allowing a more comprehensive assessment of the examined person's health status. The system automatically generates reports and alerts that can be sent to doctors or other specialists, providing immediate support and intervention. With this innovative integration of AR technology with advanced biometric monitoring, the invention represents a significant step forward in the field of neurology and remote healthcare.

These and other advantages and benefits of the invention will become more apparent based on the following detailed description.

The subject of the invention in an embodiment is shown in the drawing, in which:
Fig. 1 shows a general diagram of the structure and operation of a system according to the invention,
Fig. 2 shows a simplified block diagram of main components of a system according to the invention.

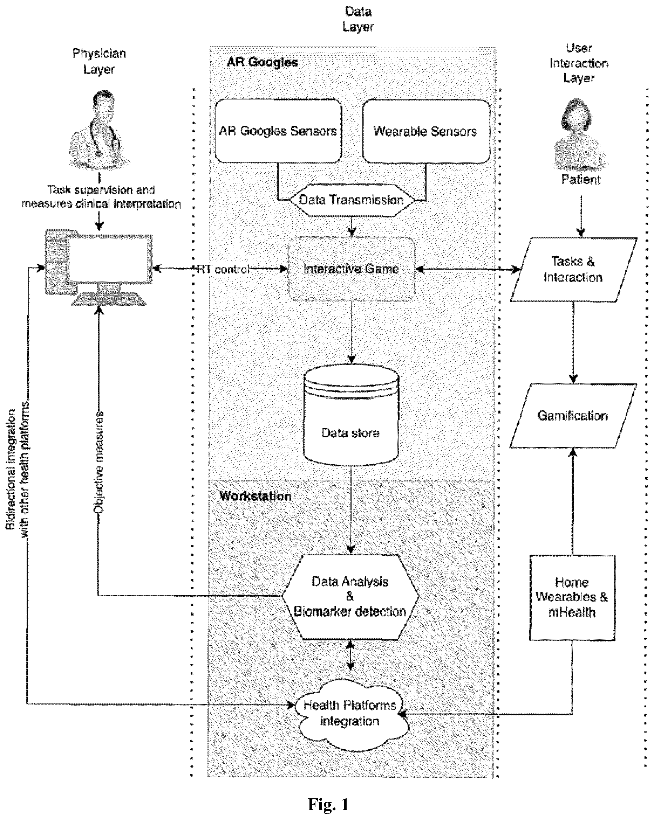

Fig. 1 shows a simplified diagram of a system for measuring and analyzing human vital and behavioral signals in Augmented Reality (AR). The system is designed to provide real-time monitoring and analysis of various physiological and behavioral parameters, particularly for neurological, neurodegenerative diseases, but is not limited to the listed applications and can be used for diagnostics of various other diseases and general health conditions.

As shown in Fig. 1, the system comprises three layers, i.e. a data layer, a user interaction layer and a doctor interaction layer. Interactions between the layers occur according to the illustrated dependency network. Referring to the data layer, in which data is collected and processed, the system comprises augmented reality (AR) goggles or mixed reality (MR) goggles or virtual reality (VR) goggles or other types of goggles of similar structure and functionality (whereas the further description will focus directly on AR goggles) containing a first set of sensors, intended to be arranged on the head of the examined person, a second set of wearable sensors intended to be placed on the body of the examined person or in the vicinity therof, a database and one or more workstations (e.g. a computer). The AR goggles constitute the basic element of the system and perform two basic functions, i.e. they serve as an interface for the examined person and as a data acquisition platform. The function of the user interface of the AR goggles relies on using a display built into the AR goggles placed on the head of the examined person to display commands, tasks or other types of indications or providing stimuli to the exmined person. The display of individual commands, tasks or indications as well as the course of the examination can be controlled by personnel supervising the examination. The data acquisition function of the AR goggles is that through the use of sensors and detectors, both integrated ones belonging to the first group and external ones belonging to the second group, which communicate with the AR goggles, synchronized data acquisition is possible from the aforementioned sensors and detectors, used to detect and estimate specific biomarkers.

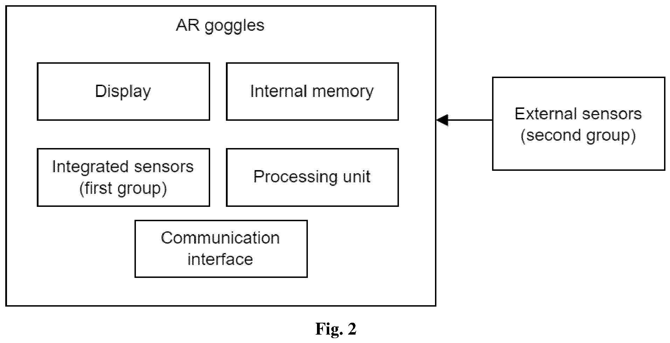

The AR goggles comprise technologically advanced components integrated in an ergonomic housing that is comfortable to wear on the head of the examined person. The key elements of the design are shown in the simplified diagram shown in Fig. 2 and include an integrated processing unit, an internal memory, one or more communication interfaces and a display integrated in the goggles. The processing unit in the system according to the invention plays a key role in collecting, analyzing and interpreting data from both the sensors of a first group integrated in the goggles and the external sensors of a second group. It receives real-time data from various sources (sensors explained in more detail later in the description) of the system according to the invention. The processing unit of the AR goggles is also used for communication with other processing units, such as e.g. a workstation, a control station, etc. and other units external to the AR goggles having appropriate communication means. The internal memory is operatively connected to the processing unit of the AR goggles and enables recording and storing of parameters recorded by the system's sensors, as well as other data, including, for example, processing results, etc. Said display is a transparent display, enabling the examined person to see the surroundings while simultaneously overlaying virtual information thereon. This display is a key element of the user interface, providing real-time commands and indications. Exemplary types of displays that may be used in the present invention include:
- transparent displays that allow virtual content to be displayed on transparent panels, allowing both the real world and additional virtual information to be seen;
- projection displays that use image projection onto special surfaces (e.g. glass or screens) or lenses to generate virtual content on the view of the surroundings;
- holographic displays that allow three-dimensional images to be generated without the use of flat screens. Using light interference, it is possible to create images with depth and three-dimensionality, allowing for more realistic display of virtual content;
- retinal displays that emit images directly onto the user's retina. This advanced technology allows virtual content to be displayed directly on the retina, providing a very natural and comfortable user experience.
- displays that use light projection principles, generating images by focusing or dispersing light in a specific way. Light projections can be used to display virtual content on various surfaces, such as glass or lenses;
- binocular displays that provide separate images for each eye of the user, which allows for the generation of a three-dimensional effect and better depth of perception.

In one preferred embodiment, the display screen includes one or more light wires that are arranged to illuminate the user and to transmit ambient light to the user. The term "light wires" in the context of augmented reality refers to a screen or goggles/glasses structure that includes such light wires. These wires are arranged to transmit an image or data to the user by being placed in the AR goggle glasses or helmet. The light wires may be made of optical fibers or other advanced materials that enable image transmission without loss of quality and with minimal light loss. They ensure that the AR user receives high quality image and information from the digital augmented reality layer that is displayed in front of his or her eyes.

The above list of possible display technologies presents only possible examples and the present invention is not limited thereto. In other words, any suitable display that will fulfill the required function and will be technically feasible to implement in goggles placed on the head of the examined person may be used in the invention.

In order to enable monitoring of health status or other parameters of the examined person, the AR goggles may be equipped with one or more of the following integrated sensors or sensing elements of the first group: RGB cameras directed at the environment around the examined person (e.g. front cameras, side cameras), enabling tracking of movements, such as e.g. tracking of the hands in order to measure pathological hand movements, the RGB cameras can be used e.g. in a limb movement segmentation and tracking system based on the analysis of images from the RGB cameras and enabling precise tracking of the examined person's movements; a microphone or a microphone array, e.g. for recording voice and speech, which is analyzed and processed e.g. in order to collect information on how and what the examined person says; IR cameras (i.e. cameras operating in the infrared range) and motion sensors used, for example, to precisely monitor eye movements, accommodation ability (enabling visual acuity at various distances) and vergence (control of the convergence of the visual axes on one object) of the examined person, a gyroscope, an accelerometer, a magnetometer (also referred to as motion sensors), which may be part of the system determining the position of the examined person in 3D space and be used to track the movement and activity level of the examined person; a heart rate sensor, which measures the heart rate and heart rate variability of the examined person based on the known principle of illuminating the skin with infrared or green light (a suitably selected LED) and analysing the reflected light, a pulse oximeter, which monitors the level of oxygen saturation in the blood (measured, for example, on the skin of the forehead or another place where the AR goggles are attached); an electrodermal activity (EDA) sensor that detects changes in skin conductance (measured, for example, on the skin of the forehead or elsewhere on the AR goggles), indicating emotional arousal or stress level; an eye movement sensor for analyzing eye movements based on IR camera streaming and computer vision algorithms; a thermometer for measuring temperature (measured, for example, on the skin of the forehead or elsewhere on the AR goggles); a light sensor (for more precise heart rate measurement, by optimizing the performance of the LEDs used for this purpose); a near infrared spectroscopy sensor (FNIRS), an electrode array together with an EEG sensor (e.g., in the form of a behind-the-ear attachment). One skilled in the art will appreciate that the sensors of the first group may also include other sensors that are not explicitly mentioned herein or sensors with equivalent functionality.

Due to the placement of the integrated sensors of the AR goggles directly in or on the housing of the AR goggles, the scope and possibilities of measuring certain important physiological or behavioral parameters of the examined person may be limited or practically impossible. Therefore, the system according to the invention may also use additional wearable sensors from the second group, which may be integrated in separate wearable devices or operate individually, whereby additional parameters, in particular physiological and/or behavioral parameters of the examined person, may be recorded, in order to enable a more precise, multimodal analysis of the health status of the examined person. Such sensors are usually placed on specific parts of the examined person's body or in its vicinity. The external sensors may have their own power source (e.g. a battery) and a wireless transmission module, so that they can be freely placed in any suitable place without the need to provide and connect additional cables. In this way, the measurement signals from the external sensors can be conveniently transmitted to another device (i.e. the AR goggles in the present invention) wirelessly. Examples of such wearable sensors include the following sensors: an electrocardiogram (ECG) sensor that records the electrical activity of the heart; a blood pressure monitor that measures blood pressure of the examined person; an electroencephalography (EEG) sensor to monitor the electrical activity of the brain; an electromyography (EMG) sensor for recording muscle activity; a GPS module to track the examined person's position; Bluetooth optionally with Proximity technology to monitor the proximity of devices; an electrodermography (EDA) sensor for recording electrodermal changes; shoe insoles for measuring foot pressure on the ground and gait analysis (based on a group of pressure sensors, e.g., in the form of a sensor array that maps pressure across the entire surface of the insole); electrodes placed on the neck for measuring vocal folds; a body temperature sensor based on thermoelectric generator technology, thermocouples, or thermistors; an acoustic signal sensor; and other wearable sensors that can make medical or auxiliary measurements, including a light sensor placed on the front of the device. Depending on the specific implementation, some of the aforementioned wearable sensors may alternatively be integrated into the AR goggles. A person skilled in the art will appreciate that the sensors of the second group may also include other wearable sensors not explicitly mentioned herein or sensors with equivalent functionality.

In the context of the present invention, "sensors" belonging to the first or second group are to be understood as any technical means enabling the detection, recording, monitoring, etc. of vital, physiological, behavioral and other parameters of the examined person relevant for monitoring the health status. Alternatively, said sensors may also be referred to equivalently as sensor devices, sensor means, sensing elements, transducers or using similar terminology.

The aforementioned sensors are placed, as required, at a suitable location on or near the body of the examined person. Within the scope of the technical implementation of the invention, the location of the individual sensors may for example be as follows:
- RGB cameras and microphones are placed directly on the AR goggles' arms, providing precise recording of the examined person's surroundings and sounds.
- heart rate sensor, ECG sensor, blood pressure sensor, light sensor, pulse oximeter sensor, and electrodermal activity sensor are integrated into wristbands worn on the examined person's wrists or chest, enabling continuous monitoring of vital signs.
- accelerometers, gyroscopes, and magnetometers are placed inside the AR goggles, close to the user's head region, enabling precise monitoring of head movements and orientation.
- IR cameras for analyzing eye movements are integrated around the user's eye region, enabling precise tracking of eye movements.
- depth cameras for analyzing the 3D structure of the face, enabling tracking of facial movements and expressions.

In the system according to the invention, sensor integration plays a key role in ensuring effective patient interaction and feedback. The main goal is to create a dynamic environment that responds to the behaviors and needs of the examined person, which enables personalized experiences and more effective healthcare.

It will also be understood that, depending on the requirements, different configurations are possible, in which individual sensors can be integrated in the AR goggles or placed independently of the AR goggles. In other words, the above enumerations are to be considered as examples and the listed sensors are not limited to the indicated use, i.e. sensors indicated as integrated in the AR goggles can be created independently of the AR goggles, and some wearable sensors can be integrated in the AR goggles as needed. Furthermore, depending on the needs, from the group of all sensors and sensors mentioned in this application, those that are most suitable for monitoring selected parameters helpful in assessing the health status of the examined person from the point of view of a given disease or in accordance with a previously defined examination profile can be selected and used.

For communication between individual components, the system according to the invention uses communication interfaces such as: WiFi, Bluetooth, Amazon Sidewalk, ANT+, Z-Wave, Zigbee, or other suitable wireless or wired interfaces, thanks to which it is possible to integrate the system with additional wearable sensors worn on the patient's body and used to record vital parameters and other relevant signals. Using the indicated communication techniques, data can be transmitted in real time or at regular intervals. As a result, the system according to the invention can record relevant parameters from a number of sensors belonging to the first and second groups in a coordinated manner.

### Procedure for using the system according to the invention

The AR goggles equipped with appropriately selected sensors are placed on the head of the examined person in such a way that the display built into the AR goggles is located in the field of view of the examined person, and other sensors or components of the AR goggles can perform assigned functions, e.g. speakers or headphones are located near the ears. Appropriately selected fastening means, e.g. elastic band or similar elements, ensure that the AR goggles adhere to the head of the examined person so that during the further procedure they remain on the head of the examined person in a fixed position. Thanks to this, the examined person can receive comprehensive information through two different communication channels: visual and auditory, and integrated sensors from the first group can record appropriate parameters.

Additional selected wearable sensors and/or other individual sensors from the second group are placed on the body of the examined person or in its vicinity in accordance with their intended use, and then the necessary connections are established between the elements of the system to ensure ongoing communication, the AR goggles and the external sensors are calibrated, and a previously defined examination procedure is initiated, e.g. using an application specially prepared for this purpose, which can be launched in the AR goggles. The aforementioned application may take the form of a set of computer program instructions, which are strored, e.g. in the internal memory of the AR goggles and are read by the processing unit (e.g. processor) of the AR goggles, which as a result carries out a previously defined procedure by appropriately controlling the individual components. As part of the aforementioned procedure, the system according to the invention, encouraging the examined person to perform subsequent tasks within the previously prepared examination scenario, during their performance, registers all available internal and external signals, typically biometric and movement data, using the applied sensors and/or sensors from the first and second groups, synchronizing their acquisition in the time domain. For this purpose, various stimuli and motor/visual and audio tasks can be used. For example, during the task, AR goggles use built-in cameras and sensors to track the patient's movements, record ambient sounds, and collect data from wearable sensors such as a pulse oximeter, accelerometer, etc.

The data collection process begins with the activation of sensors that collect information and then pre-process it, for example by filtering noise. The data is packed into small packets that contain timestamps and sensor identifiers, and then transmitted to the AR goggles using an appropriate communication protocol. In the AR goggles, data from external sensors is integrated with internal data, such as data from built-in AR sensors. Then, the integrated data is processed for analysis in the AR goggles using the processing unit, e.g. on-premise technology, which allows monitoring of the physiological and behavioral parameters of the subject. This is possible both online and offline. The data is also secured using encryption and authorization to protect the privacy of the user.

The recorded signals or data from the sensors can be saved directly in the internal memory of the AR goggles or transmitted in real time to a workstation (e.g. a computer), smartphone or so-called cloud. The transfer can take place wirelessly, for example via Bluetooth, Wi-Fi or other wireless protocols, for example as indicated above. The workstation can be a computer or other suitable device equipped with appropriate means for sending and receiving data and processing it. The transmitted sensor data is processed in the processing unit of the AR goggles, in the workstation, smartphone or other adapted device and analyzed in order to extract relevant information. The processing unit of the AR goggles can independently perform the necessary processing and analysis, i.e. external units are not necessary for the functioning of the system according to the invention, however, supplementing the system with a computer, another AR device, smartphone or other external device can be helpful in reading relevant information on an ongoing basis and communicating it to other people, e.g. a doctor or people conducting the examination. It is also possible to partially or completely perform the necessary processing and analysis in an external device, i.e. in such a case the processing unit only pre-processes the sensor signals or sends the unprocessed data to an external device (computer, smartphone, etc.), where the actual processing, analysis and collection of data about the examined person takes place.

The analysis of signals recorded by the set of sensors and/or sensing elements of the system according to the invention may include the following main steps:
- Preprocessing - including filtering, normalization and segmentation
- Feature Analysis - extraction of features in the time and frequency domain
- Event and Anomaly Detection - pattern detection, classification
- User State Assessment: assessment of health status, response to stimuli, behavioral changes
- Report and Alert Generation: generating health reports, real-time alerts
- Personalization and Adaptation - including machine learning, personalization of stimuli.

Data preprocessing may include filtering noise and artifacts, normalizing signals to a uniform range of values, and segmenting into smaller fragments for analysis of individual events.

Signal feature analysis involves identifying key signal features, such as temporal features (e.g. heart rate, response time to a stimulus), frequency features (e.g. analysis of the EEG signal spectrum) and amplitude features (e.g. maximum and minimum signal values). Advanced algorithms, including machine learning, are used to detect specific events, patterns and anomalies in the signals, identifying characteristic patterns that may indicate specific neurological or physiological conditions, and the system then assesses or classifies behavioral changes, health status and the examined person's reactions to the provided visual and auditory stimuli. Classification can specifically involve assigning signals to different categories (e.g. normal vs. abnormal health status). The results are then used to generate reports and alerts that can be sent to doctors or other users, providing immediate support and intervention if needed. Through continuous learning and adaptation, the system adapts its models based on the collected data about the examined person, allowing for more precise prediction and response to future signals. The methods used in the analysis can be both deterministic and stochastic, with particular emphasis on algorithms and methods based on machine learning and artificial intelligence mechanisms, such as neural networks, to identify or classify patterns, anomalies or trends in the data. The aim of the analysis is to detect objective parameters describing the psychophysical state of the examined person or the course of the disease based on the assessment of the examined person's response to various stimuli during the performance of tasks. The generated results can include variables such as heart rate, stress level, sleep rhythm, changes in limb movements, reactions to visual or auditory stimuli, etc.

This process provides comprehensive monitoring and analysis, allowing for a more comprehensive understanding of the examined person's condition and generating reports and alerts when alarming signals or anomalies are detected. Machine learning can be used to personalize AR experiences based on the collected data, allowing for more precise customization of interactions to the user's individual needs.

In one variant, the system can be set to determine the health status and behavioral parameters related to the examined person's response based on a norm established from a population of other users. The collection of the norm for a subset of the population can be based on criteria relevant to the examined person and their physiological measurements and reactions to stimuli and tasks. The norm can be dynamically changed by measuring values from the sensors integrated in the goggles and the external sensors determining the user's health status and behavioral parameters related to the response.

The processed data is then integrated into the augmented reality display built into the AR goggles, overlaying the information onto the smart glasses/goggles worn by the subject. The AR visualization can include real-time graphs, graphics, avatars, or other visual indicators related to the measured vital signs and behaviors of the examined person.

The AR system allows the user to interact with the displayed information. For example, he or she can perform gestures or voice commands to access specific details, switch between different sensor readings, or set personalized alerts based on predefined thresholds. Additionally, the system can generate notifications or alerts if any vital sign or behavior exceeds a predefined threshold. This can be helpful in situations where immediate attention or intervention is required.

Depending on the requirements, the system of the invention records and stores the collected data in the internal memory of the AR goggles and/or the smartphone or in the cloud for further analysis to enable long-term monitoring, tracking progress or identifying patterns over time.

As shown in Fig. 1, the AR system has the ability to integrate with existing healthcare platforms or electronic medical records systems. This allows healthcare professionals to remotely access and review the data, allowing remote monitoring of the examined person or timely interventions.

In order to make the invention more understandable, exemplary use scenarios of the system according to the invention are presented below. It will be understood that each of the presented scenarios represents a specific variant of the system and method of the invention. In other words, the individual scenarios are based on the selection of appropriate sensors from the first and second groups and the above-described procedure for using the system is carried out for them.

### Scenario 1: Recognizing and monitoring of neurodegenerative diseases:

Initially, in the system according to the invention, the AR goggles should be configured to enable data acquisition from the first group of built-in sensors and integration with multimodal analysis software, which can be run directly in the AR goggles or optionally on an external device communicating with the AR goggles. The system should include IR cameras (operating in the infrared range) used in eye tracking modules, at least one microphone for recording speech, an accelerometer, a gyroscope, a magnetometer for measuring posture and for monitoring gait, RGB cameras for tracking upper limb movement, and depth cameras for tracking facial movements and expressions. The individual sensors from the first group are placed in a suitable location on or in the AR goggles to enable recording of relevant features of the examined person. The IR cameras and depth cameras are therefore, for example, attached to the goggles housing on the front side and oriented in the direction of the examined person's eyes. The accelerometer, gyroscope, magnetometer can be combined into a unit that is mounted to the rigid structure of the AR goggles (or integrated into it), e.g. to the housing, so that they can record the movement parameters of the examined person while wearing the AR goggles. The microphone is placed, for example, in the part of the goggles housing that is close to the mouth of the examined person, to record the voice of the examined person without any obstacles. The RGB cameras can be placed on the arms of the AR goggles and oriented downwards so that the image they record includes the upper limbs of the examined person.

In the system according to the invention, external sensors selected from the second group are also configured, such as: an electroencephalographic (EEG) sensor, an electromyographic (EMG) sensor, a GPS module, an electrodermal activity (EDA) sensor, pressure sensors integrated in shoe insoles for measuring foot pressure on the ground and gait analysis, electrodes placed on the neck for examining the vocal folds, a sensor for measuring blood pressure, heart rate, a sensor for measuring sleep quality.

Then, the system settings should be adjusted to the specific diagnostic needs related to specific neurodegenerative diseases, such as Parkinson's disease, multiple sclerosis or amyotrophic lateral sclerosis. This involves introducing a task protocol to be displayed on the AR goggles display for the examined person, which should include clear commands, with instructions including graphical representation, video presentation, audio-visual examples and sound, e.g. using a speaker or headphones built into the AR goggles.

In order to analyze eye movements, it is necessary to program eye tracking algorithms that can detect characteristic movement patterns, such as nystagmus or slowed eye reactions. Then, it is necessary to activate the acquisition of signals from the IR cameras tracking eye movements.

Speech analysis recorded using one or more microphones may include detection of changes in tempo, intonation and voice quality, which may indicate progressive neurological disorders. Speech analysis may include linguistic analysis after processing signals by automatic speech recognition algorithms and acoustic analysis.

Posture measurement may include monitoring changes in body balance and stability by analyzing data from accelerometer and gyroscope sensors.

Assessment of cognitive impairments can be performed by analyzing the examined person's response to specific visual or auditory stimuli and by assessing cognitive functions such as memory, attention and executive functions. The analysis will include signals from multiple sensors: cameras recording eye movements, speech recorded with a microphone/microphones, posture analysis (accelerometer, gyroscope, magnetometer) and upper limb movement (RGB cameras tracking the environment with algorithms for segmenting arms, hands and fingers).

Gait monitoring may include analysis of steps, pace, symmetry and gait stability using data from the accelerometer and gyroscope.

Analysis of blood pressure and pulse measurements may be recorded using a wristband or a watch placed on the wrist. Blood pressure measurement may include measurement of systolic and diastolic blood pressure. Pulse measurement is performed using a plethysmographic sensor (PPG) (using infrared and green radiation, e.g. with the use of an appropriately selected LED diode).

The previously mentioned external sensors (second group) play an important role in assessing various aspects related to neurodegenerative diseases.

The electroencephalography (EEG) sensor plays an important role in the study of brain activity. Each neurodegenerative disease can cause characteristic changes in alpha brain waves. For example, Alzheimer's disease can be associated with reduced alpha and beta wave frequency and increased theta and delta wave activity, which reflects changes in the cerebral cortex, for Parkinson's disease EEG can help assess cognitive function and detect sleep disorders, which are common among Parkinson's patients, increased delta wave activity can occur, in particular in the advanced stages of the disease, and increased theta wave activity can also be observed, in particular in the relaxation state or early stages of sleep. These patients may also show reduced alpha wave activity, in particular in the resting state with closed eyes. Additionally, beta wave activity may be reduced in Parkinson's patients, which can affect their ability to perform complex cognitive tasks. For multiple sclerosis, EEG can be used to monitor changes in brain function in response to treatment and disease progression. Increased theta wave activity may occur in these patients. Increased theta wave activity may be associated with memory problems, in particular working and episodic memory. It may also suggest difficulties with concentration and attention. Gamma wave activity may be reduced in patients with multiple sclerosis, which is associated with higher cognitive functions.

The electromyography (EMG) sensor plays an important role in assessing muscle and nerve function. Neurodegenerative diseases such as Parkinson's disease, multiple sclerosis, amyotrophic lateral sclerosis (ALS), and others often affect muscle and nerve function, which can be effectively monitored using EMG. An example of an application in Parkinson's disease: EMG can monitor tremors, muscle stiffness, and bradykinesia (slowness of movement).

The GPS module in the context of monitoring neurodegenerative diseases plays an important role in tracking and analyzing patients' movement patterns, which can provide valuable information regarding their mobility, safety, and quality of life. An example of an application in Parkinson's disease: the GPS sensor can monitor patients' movement patterns, such as the frequency and length of walks. In Alzheimer's disease, a GPS sensor can ensure patient safety by tracking their real-time location.

In the context of monitoring neurodegenerative diseases, the electrodermal activity (EDA) sensor can provide valuable information about patients' emotional state, their response to stress, and their overall mental well-being. Application example in Parkinson's disease: patients may experience increased levels of anxiety and stress, in particular in advanced stages of the disease, in Alzheimer's disease the EDA sensor can help monitor stress and anxiety levels in patients who often experience confusion and anxiety.

In the context of neurodegenerative diseases, the neck electrodes for vocal fold examination (EGG, electroglottography) can provide valuable information on muscular and neural functions related to speech and swallowing, including information on the strength, coordination, and synchronization of the vocal muscles. EGG changes in Parkinson's disease include increased phonation instability (phonation instability is often observed in Parkinson's disease, which can manifest as variability in the EGG signal during speaking), reduced signal amplitude (hypophonia leads to a decrease in the EGG signal amplitude, which is the result of weaker vocal fold activity), asymmetry of vocal fold closure (EGG may show asymmetry in vocal fold closure, which is associated with impaired muscle coordination). Other examples of the use of EGG analysis in Alzheimer's disease include increased variability in phonation cycles (in Alzheimer's disease, there may be irregularity in phonation cycles, which is visible as variability in the EGG signal), decreased signal coherence (problems with muscle coordination may lead to less coherent EGG signals, reflecting difficulties in maintaining fluency of speech), and prolonged opening and closing phases (EGG may show prolonged opening and closing phases of the vocal folds, suggesting difficulties in precise control of these muscles).

Sleep quality analysis includes collecting data on periods of light, deep and REM sleep, wakefulness time and assessing sleep quality based on this data. Sensors in the wristband or watch may monitor body movements, respiratory rate and other parameters which are analysed to determine the quality of the examined person's sleep and what the sleep patterns are. The term "sleep sensor" in the context of the present invention should therefore be understood to mean one selected sensor or a combination of sensors recording parameters related to the examined person 's sleep, including, for example, an accelerometer measuring body movements, including hand and body movements during sleep (to determine when and how intensely the examined person is sleeping and whether there are periods of restful sleep and movements, where movements may indicate periods of light, deep or REM sleep); a light-sensitive sensor (ambient light intensity, e.g. darkness, which is important for assessing sleep quality); a heart rate monitor (heart rate measurement may provide information on heart rate during different sleep stages, changes in heart rate may indicate transitions between sleep stages such as light, deep and REM sleep); a skin temperature sensor (skin temperature changes during different sleep stages, which may be used to assess sleep quality); a blood oxygen saturation (SpO2) sensor (blood oxygen saturation measurement may also be used for sleep analysis, low SpO2 levels may indicate abnormal breathing during sleep, which may affect sleep quality); or a noise sensor (e.g., to determine whether ambient noise is affecting sleep quality). In the case of including sleep analysis, one selected sensor or a combination thereof (a "sleep sensor") may be placed at an appropriate location on or near the examined person's body during sleep to take the necessary measurements, and then upon waking up the examined person puts on AR goggles and other wearable sensors, and the method of the invention is performed, wherein the final analysis of the health status takes into account the measurement data collected during sleep.

The measurement data acquired by the first group of sensors and the second group of sensors are collected, processed and subjected to joint analysis, which is the so-called multimodal analysis and is carried out in accordance with the procedure described earlier. The multimodal data analysis allows for a comprehensive assessment of the patient's condition, integrating information from various sources, such as eye movement, speech, posture, upper limb movement and gait. The analysis results can help identify characteristic features and changes related to a specific neurodegenerative disease, which will allow doctors to diagnose and monitor the progression of the disease more quickly and accurately.

For example, eye movement analysis can detect characteristic patterns that indicate the presence of Parkinson's disease or multiple sclerosis. Speech analysis can reveal changes in articulation, tempo and rhythm that are typical for neurological diseases such as amyotrophic lateral sclerosis. Posture measurement can help assess the risk of falls and monitor the progression of a neurodegenerative disease, e.g. by detecting an increase in postural instability. Assessment of cognitive functions can provide information about the progression of the disease and help adjust the treatment plan and support the patient. Gait analysis may enable the detection of early symptoms of movement disorders and monitoring the effectiveness of therapy. Additionally, the system of the invention may analyze data related to blood pressure and pulse measurement, which are important for monitoring cardiovascular health and in the context of monitoring neurodegenerative diseases. Regular blood pressure and pulse measurements may help to identify changes that may be associated with the progression of diseases such as Parkinson's disease or multiple sclerosis. The measurement results may provide information on hemodynamic stability and may serve as an additional health indicator that supports the overall assessment of the health status of the examined person in the context of his neurological disease. Sleep quality analysis may be crucial for monitoring patients with neurodegenerative diseases, as it may include the assessment of light, deep and REM sleep periods and the identification of insomnia episodes. The importance of full-quality sleep for brain health is well documented, and data from sleep quality analysis may provide information on possible sleep disorders that may accompany neurodegenerative disease. Regular assessment of sleep quality may help to better manage symptoms and improve the patient's quality of life.

As a result, the system and the method according to the invention in the presented configuration allow to determine whether the examined person has significant symptoms related to a neurodegenerative disease or not, and, if they are present, allows to deduce the progression or degree of advancement of the disease. The invention does not cover the diagnosis itself, which is made directly by a doctor, who can remotely read the results of the analysis obtained thanks to the system and method according to the invention and make the appropriate assessment on this basis. In this connection, it should be clearly indicated that the described method is not a diagnostic method, but a method for monitoring health status, which, thanks to multimodal analysis, provides significant information concerning the health of the examined person.

### Scenario 2: Monitoring heart diseases (e.g. heart failure):

The AR goggles can be configured with sensors that enable measurement of parameters related to heart function, such as heart rate (remote photoplethysmography, obtained from analysis of IR signals observing the upper part of the user's face), electrical activity of the heart (ECG, sensor placed on the chest), blood pressure (sensor placed on the user's wrist), blood oxygen saturation (pulse oximetry, sensor placed on the user's wrist), measurement of the amount of light falling on the surface of the light sensor (for more precise measurement of heart rate, by optimizing the operation of LED diodes), monitoring of gait and motor activity (accelerometer, gyroscope, magnetometer). The system can be adapted to monitor patients with heart failure (HF) by tracking changes in these parameters and recording them in real time. The system configuration should also include data analysis algorithms that are able to interpret measurements from the sensors and provide the user or doctor with information on the condition of the patient's heart.

Monitoring the heart rate and rhythm allows for the detection of irregularities in the heart, such as arrhythmias, which may be a symptom of heart failure. Measuring the electrical activity of the heart (ECG) allows for a more accurate assessment of the heart rhythm and identification of potential conduction disorders. Blood pressure measurement allows for the assessment of systolic and diastolic pressure and monitoring of its changes, which is important in the control of heart failure. Monitoring the oxygen saturation of the blood (pulse oximetry) can provide information on the degree of blood oxygen saturation and lung function, which is important in the case of heart failure. Analysis of data from these parameters can enable early detection of deterioration of the patient's condition, allowing for a quick response and adjustment of therapy. Using the AR goggles to present data can allow the doctor or medical staff to conveniently access information about the heart condition of the examined person in real time, which can increase the efficiency of monitoring and managing the care of the examined person.

### Scenario 3: Detecting symptoms of myocardial infarction and stroke

The AR goggles can be configured with a set of sensors that allow for the collection of data related to brain function, such as eye movements, speech, reactions to visual and auditory stimuli, and physiological parameters such as blood pressure and heart rate. Data analysis algorithms should be implemented in the system that are able to identify characteristic symptoms of stroke, such as asymmetry of eye movements, speech disorders, loss of balance, and others. Additionally, the AR goggles can be equipped with communication functions with medical systems, allowing data to be sent to the doctor or medical staff for immediate assessment. A patient who suspects that he or she may be having a stroke puts on the AR goggles, which immediately begins analyzing physiological parameters and brain function. The system monitors the patient's eye movements, looking for asymmetries or abnormal patterns that may indicate a stroke. The AR goggles also present the patient with specific visual or auditory stimuli and then monitor their reactions, looking for perceptual or communication disorders that may be symptoms of a stroke. Meanwhile, the system monitors physiological parameters such as blood pressure and heart rate, which can provide additional information about the patient's condition. Based on the collected data, the data analysis algorithms in the AR goggles can automatically identify a suspected stroke and send a notification to the doctor or medical staff, allowing for quick intervention and treatment adjustment. By immediately recognizing a stroke using the AR goggles, the patient can be quickly directed to the appropriate medical care, increasing the chances of quick and effective treatment and minimizing the damage resulting from the stroke.

In another embodiment, the system of the invention may be configured to present a stimulus by displaying a plurality of images to a user via the display of the AR goggles, wherein one of the images is located in a different depth plane than the others. The evaluation of the examined person's reaction consists in this example in measuring the accommodation, vergence state and/or other reactions of the user's efferent system of the eyes (processes controlling eye movements in response to external and internal stimuli), measuring the abrupt eye movements that allow for rapid shifting of the gaze from one point to another visual target (visual saccades), measuring the user's points of interest in relation to the presented content (e.g. a clock with hands set to a specific hour), and determining the image perceived by the user by matching the measured accommodation, vergence state (the ability of the eyes to focus on one object by converging their visual axes, i.e. directing the eyes in one direction to look at the same point in space) and/or other reactions of the efferent system to the expected accommodation and/or vergence state for one of the images or the other of the images.

As described above in the exemplary scenarios, the developed invention has great potential in the field of medicine and health and significantly improves the quality of diagnostics and health care. This is due to the synchronized recording of signals from various sensors (e.g. infrared cameras, RGB cameras, microphones, IMU sensors (accelerometer, gyroscope, magnetometer) and other additional external sensors, which allows for a specific acquisition of many signals (multimodal) monitoring human behavior while using augmented reality technology. Such synchronization and analysis allows for a comprehensive objective assessment of the user during the implementation of defined scenarios.

## Claims

1. A system for monitoring health status of an examined person, comprising:
- goggles designed to be mounted on the head of the examined person and comprising:
-- a transparent display positioned in the field of view of the examined person, adapted to enable simultaneous observation of the surroundings and displaying predetermined augmented reality virtual content at different depth levels;
-- a processing unit connected to an internal memory;
-- one or more sensors selected from a first group of sensors integrated with the goggles for monitoring physical and/or behavioral parameters of the examined person; and
-- at least one communication interface;
**characterized in that** it further comprises:
- one or more sensors selected from a second group of sensors for monitoring physical and/or behavioral parameters of the examined person, arranged on or near the body of the examined person, communicatively connected to the goggles by means of said at least one communication interface, the sensors of the second group being independent of the sensors of the first group;
wherein the goggles are in particular augmented reality (AR), mixed reality (MR) or virtual reality (VR) goggles and are configured to provide predetermined instructions or stimuli to the examined person, to collect and store sensor signals from the first group of sensors and the second group of sensors in the internal memory, reflecting the response to the instructions or stimuli, and to jointly process and multimodally analyze them using the processing unit in order to assess the health status of the examined person.

2. The system according to claim 1, **characterized in that** the first group of sensors integrated in the goggles comprises in particular RGB cameras directed at the environment around the examined person, a microphone or a microphone array, infrared cameras directed at the eyes of the examined person; a heart rate sensor; a blood pressure monitor; a pulse oximeter; an electrodermal activity sensor; an accelerometer, a gyroscope and a magnetometer; an eye movement sensor; a thermometer; depth cameras, an infrared (IR) sensor; a near infrared spectroscopy sensor (FNIRS); an electrode array together with an EEG sensor.

3. The system according to claim 1, **characterized in that** the second group of sensors comprises in particular an ECG sensor, an EEG sensor; an EMG sensor; a GPS module; an EDA sensor; pressure sensors integrated in shoe insoles for measuring foot pressure on the ground and for analyzing gait; electrodes placed on the neck for measuring the vocal folds, a blood pressure sensor, a heart rate sensor (PPG), a light sensor, a temperature sensor, an acoustic signal sensor, an accelerometer, a gyroscope, a magnetometer, a blood oxygen saturation sensor.

4. The system according to claim 1, **characterized in that** the goggles are additionally equipped with at least one loudspeaker or headphones.

5. The system of claim 1 or 4, **characterized in that** the display of the goggles is configured to provide the instructions or stimuli in a visual form and/or the speaker or headphones are configured to provide the instructions or stimuli in an audible form, wherein the visual instructions or stimuli are in the form of one or more images at different depth levels.

6. The system according to any one of the preceding claims, **characterized in that** the system is configured to recognize and monitor neurological or neurodegenerative diseases, wherein:
- the sensors used from the first group include: IR cameras for tracking eye movement, at least one microphone for recording speech, an accelerometer, a gyroscope and a magnetometer for measuring posture and monitoring gait, RGB cameras for tracking upper limb movement and depth cameras for tracking facial movements and expressions,
- the sensors used from the second group include an EEG sensor; an EMG sensor; a GPS module; an EDA sensor; pressure sensors integrated in shoe insoles for measuring foot pressure on the ground and gait analysis; electrodes placed on the neck for examining vocal folds, a blood pressure sensor, a heart rate sensor (PPG), a temperature sensor, a blood oxygen saturation sensor.

7. The system according to any one of the preceding claims, **characterized in that** the system comprises at least one external workstation communicatively connected to the goggles via said communication interface, in particular a computer or a smartphone, for receiving, processing and/or collecting sensor data and/or signals.

8. A method for monitoring health status of an examined person, **characterized by:**
- mounting, on the head of the examined person, goggles equipped with a transparent display, in particular augmented reality (AR), mixed reality (MR) or virtual reality (VR) goggles, and equipped with one or more integrated sensors selected from a first group of sensors for monitoring physical and/or behavioral parameters of the examined person;
- arranging, on the body of the examined person or in its vicinity, one or more sensors selected from a second group of sensors for monitoring physical and/or behavioral parameters of the examined person, the sensors of the second group being independent of the sensors of the first group;
- providing predetermined visual and/or auditory instructions or stimuli to the examined person by means of a transparent display or by means of at least one loudspeaker or headphones;
- recording, by means of sensors from the first group and sensors from the second group, physiological and/or behavioral parameters reflecting the reaction of the examined person to the provided instructions or stimuli;
- jointly analyzing the recorded sensor data, wherein the analyzing includes:
-- pre-processing that includes filtering, normalization, and segmentation of measurement signals;
-- analysis of time and/or frequency and/or amplitude features of the pre-processed signals in order to extract significant features;
- detection of patterns and classification of the extracted features;
- assessment of the health status of the examined person based on the performed classification of features.

9. The method according to claim 8, **characterized in that** the first group of sensors integrated in the goggles, which are used for recording physiological and/or behavioral parameters of the examined person, comprises in particular RGB cameras directed at the environment around the examined person, a microphone or a microphone array, cameras operating in the infrared range directed at the eyes of the examined person; a heart rate sensor; a blood pressure monitor; a pulse oximeter; an electrodermal activity sensor; an accelerometer, a gyroscope and a magnetometer; an eye movement sensor; a thermometer, depth cameras, an infrared (IR) sensor; a near infrared spectroscopy sensor (FNIRS); an electrode array together with an EEG sensor.

10. The method according to claim 8, **characterized in that** the second group of sensors which are used for recording physiological and/or behavioral parameters of the examined person, comprises in particular an ECG sensor, an EEG sensor; an EMG sensor; a GPS module; an EDA sensor; pressure sensors integrated in shoe insoles for measuring foot pressure on the ground and for analyzing gait; electrodes placed on the neck for measuring the vocal folds, a blood pressure sensor, a heart rate sensor (PPG), a light sensor, a temperature sensor, an acoustic signal sensor, an accelerometer, a gyroscope, a magnetometer, a blood oxygen saturation sensor.

11. The method according to claim 8, **characterized in that** machine learning and artificial intelligence algorithms, in particular neural networks, are used for detecting patterns and classifying the extracted features.

12. The method according to any one of claims 8 - 11, **characterized in that** based on the sensor signals and data collected for a given examined person a model is created using machine learning to personalize and adapt the analysis.

13. The method according to any one of claims 8 - 12, **characterized in that** based on the assessment of the health status of the examined person, feedback, health reports or alerts are generated in real time and displayed on the display of the goggles and/or transmitted to one or more external devices.

14. Method according to any one of claims 8-13, **characterized in that** the method is adapted for monitoring neurological or neurodegenerative diseases, wherein:
- physiological and/or behavioral parameters are recorded using integrated sensors from the first group: eye movement using IR cameras, speech using at least one microphone, posture and gait using an accelerometer, a gyroscope and a magnetometer, movement of the upper limbs using RGB cameras and facial movements and expressions using depth cameras;
- physiological and/or behavioral parameters are recorded using sensors from the second group: brain activity using an EEG sensor; muscle and nerve function using an EMG sensor; movement patterns using a GPS sensor; electrodermal activity using an EDA sensor; foot pressure on the ground and gait using pressure sensors integrated in shoe insoles; vocal fold activity using electrodes placed on the neck, blood pressure using a blood pressure sensor, heart rate using a PPG sensor; body temperature using a temperature sensor; blood oxygen saturation using a blood oxygen saturation sensor.
wherein said multimodal analysis inclues jointly:
- analysis of eye movement in order to detect characteristic movement patterns, in particular nystagmus and/or slowing of eye reactions;
- analysis of speech, in particular tempo, intonation and voice quality;
- analysis of posture measurement, in particular changes in body balance and stability;
- analysis of the examined person's reaction to visual and/or auditory stimuli;
- analysis of facial expressions;
- analysis of gait;
- analysis of electroencephalographic (EEG) signals;
- analysis of the near infrared spectroscopy signal carrying diagnostic information regarding active parts of the brain;
- analysis of the electromyography (EMG) signal carrying information about muscle activity;
- analysis of signals from electroglottography, which provides information about vocal fold activity;
- analysis of signals carrying information about systolic and diastolic blood pressure;
- analysis of the photoplethysmographic signal, providing information about heart rate;
- analysis of sleep quality based on one or more parameters measured during the sleep of the examined person, including: body movements, heart rate, skin temperature, blood oxygen saturation, ambient light intensity.

15. A computer-readable storage medium comprising computer program instructions strored thereon which, when executed by a processing unit, perform the method of any one of claims 8-14.
